# EUROPÄISCHE PATENTANMELDUNG

(11) **EP 1 652 933 A1**
(43) Veröffentlichungstag der Anmeldung: **03.05.2006**
(21) Anmeldenummer: 04025361.9
(22) Anmeldetag: 26.10.2004
(51) Int. Cl.: C12Q 1/02

(54) **Verfahren zum Bestimmen der ökotoxikologischen Wirkung feststoffadsorbierter und/oder im Wasser gelöster Schadstoffe in einer Matrix und Testkit zum Durchführen dieses Verfahrens**

(71) Anmelder: Dr. Fintelmann und Dr. Meyer Handels- und Umweltschutzlaboratorien GmbH, 22761 Hamburg (DE)
(72) Erfinder: Neumann-Hensel, Helga, Dr., 21077 Hamburg (DE)
(74) Vertreter: Fleck, Thomas

(57) **Zusammenfassung**

Die vorliegende Erfindung betrifft ein Verfahren zum Bestimmen der ökotoxikologischen Wirkung feststoffadsorbierter und/oder in Wasser gelöster Schadstoffe in einer Matrix mittels Zusatz reaktivierter, vorher gefriergetrockneter Bakterien als Inokulum, Inkubation desselben und Ermittlung der Hemmung ihrer Dehydrogenaseaktivität, die über den Umsatz eines Redoxfarbstoffes bestimmt wird. Die vorbekannten Verfahren dauern länger als einen Arbeitstag und sind relativ aufwendig. Der Erfindung liegt deshalb die Aufgabe zugrunde, dieses Verfahren schneller mit geringem Arbeitsaufwand zu machen und einen praktischen Testkit zu schaffen. Diese Aufgaben werden erfahrungsgemäß dadurch gelöst, dass der Testansatz in Mikrotiterplatten durchgeführt wird, wobei die Matrix in einer Menge von < 1g eingegeben wird, und wobei die Inkubation in einem Schüttler/Mischer durchgeführt wird, und wobei die Zunahme des reduzierten Redox-Stoffes fluorimetrisch direkt in der Mikrotiterplatte erfasst wird, und dass sämtliche Verfahrensschritte in einer Zeitspanne < 8 Std. durchgeführt werden bzw. durch einen Testkit nach Anspruch 11.

## Beschreibung

Die Erfindung betrifft ein Verfahren nach dem Oberbegriff des Anspruches 1 und ein Testkit zum Durchführen des Verfahrens.

Vorbekannte Verfahren bauen auf dem Feststoffkontakttest nach DIN 38412-48 auf. Dieser Test ist sehr zeit- und arbeitsaufwendig und dadurch für die tägliche praktische Anwendung nicht geeignet.

Das Prinzip des Verfahrens zur Bestimmung der direkten Bodentoxizität ist die Ermittlung der Hemmung der Dehydrogenaseaktivität zugesetzter Testbakterien in der zu untersuchenden Matrix, wie Boden oder Sediment, bezogen auf eine unkontaminierte Referenzmatrix.

Zur klassischen Testdurchführung nach DIN werden mindestens 3 g Boden in verschließbare Zentrifugenröhrchen eingewogen. Es werden jeweils drei Parallelen für den Testansatz mit Bakterien (=Probe) und den Testansatz ohne Bakterien (Blindwert) angesetzt. Nach Zugabe von 3 ml vollentionisiertem Wasser werden die Ansätze im Wasserbad pasteurisiert. Anschließend werden 2 ml Inokulum bzw. 2 ml Nährmedium für die Blindwerte hinzupipettiert. Die Inkubation erfolgt 2 Stunden im Dunkeln bei 30°C in einem Überkopf-Schüttler. Nach Zugabe von 4 ml Resazurinlösung werden die Ansätze erneut im Überkopf-Schüttler 40 min lang inkubiert. Um die Reaktion abzubrechen, werden die Gefäße bei Raumtemperatur 5 min bei 2000 g zentrifugiert und anschließend in ein Eisbad gestellt. Die Ermittlung der Dehydrogenaseaktivität erfolgt über die Bestimmung des Umsatzes des Redoxfarbstoffes Resazurin. In Anwesenheit von Dehydrogenasen wird Resazurin in Abhängigkeit von der enzymatischen Aktivität zu Resorufin reduziert. Das blaugefärbte Resazurin besitzt ein abweichendes Absorptionsmaximum vom rosagefärbten Resorufin, so dass das Resazurin photometrisch bei 600 nm bestimmt wird. Hierzu muss der Boden bzw. das Sediment abgetrennt werden, so dass die Messung im trübungsfreien Filtrat erfolgen kann. Der zeitliche Aufwand des Filtrierens jedes Testansatzes ist sehr groß und arbeitsintensiv.

Die Anzucht der Testbakterien, d.h. der Vorkultur, muss einen Tag vor der Testdurchführung begonnen werden. Hierzu werden tiefgefrorene Bakterien der Stammkultur schonend aufgetaut und 50 ml Nährmedium mit 0,1 ml der Bakteriensuspension angeimpft. Die Kultur muss 14 bis 16 Stunden über Nacht im Brutschrank unter Lichtausschluss mit einer Frequenz von 150 min⁻¹ schütteln. Zur Herstellung des Inokulums wird die Vorkultur am nächsten Morgen zunächst mit Nährmedium auf eine optische Dichte (OD₆₀₀) von 0,2 eingestellt und unter den oben beschriebenen Bedingungen erneut für 1,5 bis 2 Stunden inkubiert. In dieser Zeit muss eine optische Dichte von 0,4 erreicht werden. Hierbei soll überprüft werden, ob sich die Bakterien in der exponentiellen Wachstumsphase befinden, d.h. ob sie sich mit maximaler Wachstumsrate vermehren. Das vorbekannte Verfahren dauert insgesamt mindestens 20-24 Stunden.

Der vorliegenden Erfindung liegt deshalb die Aufgabe zugrunde, das eingangs genannte Verfahren derart zu verbessern, dass es schnell und ohne großen Arbeitsaufwand mit einem praktischen Testkit durchgeführt werden kann. In überraschender Weise wird diese Aufgabe durch das erfindungsgemäße Verfahren gemäß Anspruch 1 bzw. durch den Testkit gemäß Anspruch 11 gelöst. Dieses erfolgt in Mikrotiterplatten und unterscheidet sich gravierend von dem vorbekannten Testverlauf. Insbesondere nimmt es nur 8 Std. oder weniger, vorteilhafterweise insgesamt nur 4 bis 6 Std. in Anspruch.

Die erfindungsgemäße Feststoffeinwaage beträgt < 1 g, vorteilhafterweise 0,6 g, wobei alle Volumina der weiteren Zusätze derart reduziert werden, dass die Verhältnisse im Vergleich zum vorbekannten Verfahren erhalten bleiben. Die Inkubation erfolgt nicht am Überkopf-Schüttler, sondern auf einem Horizontalschüttler.

Zur Durchführung des erfindungsgemäßen Verfahrens wird der Feststoffkontakttest in Mikrotiterplatten vorgenommen. An Stelle der photometrischen Messung der Substratabnahme wird erfindungsgemäß die Produktzunahme des reduzierten Redoxstoffes fluorimetrisch erfasst. Diese Messung kann in der Bodensuspension, d. h. direkt in der Mikrotiterplatte erfolgen. Es entfallen somit die Schritte der Zentrifugation und der Filtration, was einen erheblichen Beitrag zur Zeitersparnis darstellt. Durch eine Messung der relativen Fluoreszenz im Abstand von 15 min wird vorteilhafterweise eine Kinetik aufgenommen, die gegenüber der Endpunktbestimmung weitergehende Informationen liefert.

Als Testorganismen kommen u.a. hier gefriergetrocknete (lyophilisierte) Bakterien der Gattung *Arthrobacter globiformis* oder ähnliche zum Einsatz, wobei auf eine Übernachtkultivierung verzichtet werden kann. Die Bakterien werden mit einer geeigneten Reaktivierungslösung reaktiviert und sind sofort für den Einsatz im Feststoffkontakttest bereit. Neben einer Zeitersparnis ist ein weiterer Vorteil die einfache Handhabung und Konservierung der gefriergetrockneten Bakterien.

Im folgenden werden beispielhafte Angaben zum erfindungsgemäßen Verfahren gemacht, das unter Verwendung des erfindungsgemäßen Testkits im Laboreinsatz durchgeführt wird, worauf die Erfindung verständlicherweise nicht beschränkt ist:
Durchführung des Feststoffkontakttests mit gefriergetrockneten Bakterien in Mikrotiterplatten am Beispiel einer Chemikalienprüfung mit Benzalkoniumchlorid (BAC)

### Vorbereitung des zu untersuchenden Bodens

Kontamination des Referenzbodens St.2.2 zur Untersuchung der Toxizität der eingesetzten Testsubstanz

Das Testsubstrat wurde einen Tag vor Beginn des Tests kontaminiert. Der Konzentrationsbereich des BAC sollte zwischen 400 und 800 mg/kg Frischgewicht Boden liegen. Hierzu wurde eine Stammlösung der Testsubstanz mit einer Konzentration von 10 mg/ml angesetzt. Es wurde eine Verdünnungsreihe hergestellt (siehe Tabelle 1) und von diesen verdünnten Chemikalienlösungen wurde jeweils 1 ml auf 10 g Boden gegeben und gründlich homogenisiert.

**Tabelle 1: Kontamination des Referenzbodens**

| **Konzentration kontaminierter Boden** | **Volumen Stammlösung (10mg/ml) Lösung I** | **Volumen H**_{**2**}**O Lösung II** | **Konzentration der BAC-Lösung (Lösung I+II) Lösung III** | **Konzentration in 10g Boden bei Zugabe von 1ml Lösung III** |
|---|---|---|---|---|
| [mg/kg] | [ml] | [ml] | [mg/ml] | [mg/g] |
| 400 | 4 | 6 | 4 | 0,4 |
| 500 | 5 | 5 | 5 | 0,5 |
| 600 | 6 | 4 | 6 | 0,6 |
| 750 | 7,5 | 2,5 | 7,5 | 0,75 |

Für den Kontrollansatz wurde 1 ml VE-Wasser appliziert. Durch die applizierte Lösungsmenge wurde eine Wasserhaltekapazität (WHK) von 40 - 60% eingestellt. Der in Bechergläsern abgefüllte kontaminierte Boden wurde abgedeckt und für 16 bis 18 Stunden bei Raumtemperatur im Dunkeln zur Gleichgewichtseinstellung belassen.

### Einwaage in Mikrotiterplatte

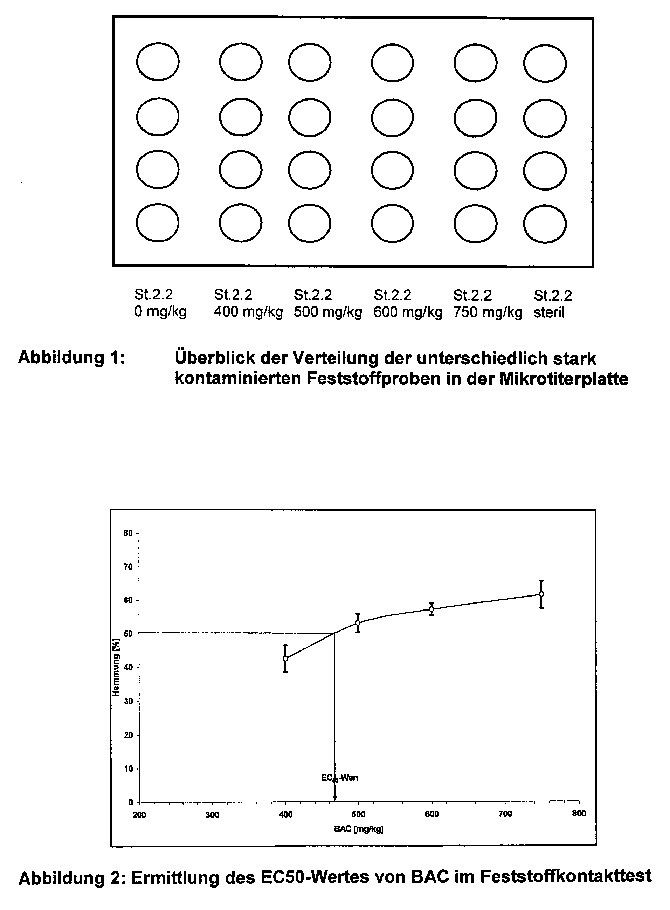

Die Mikrotiterplatte hat 24 Vertiefungen. Die Einwaage der unterschiedlich stark kontaminierten Feststoffproben erfolgte nach folgendem Schema (siehe Abb. 1).

Je Vertiefung werden 0,6 g Boden eingewogen. In der ersten Reihe wurde der unkontaminierte Referenzboden von oben nach unten eingewogen. In die vier darauf folgenden Reihen wurden die kontaminierten Böden mit steigender Konzentration gegeben. Die letzte Reihe dient als Sterilkontrolle, hier wurde ebenfalls unkontaminierter Boden eingewogen, welcher im späteren Verlauf des Versuches nicht mit den Testbakterien angeimpft wird, sondern mit dem Nährmedium B. Es wurden vier Parallelwerte bestimmt.

### Pasteurisierung des Bodens

Zur Inaktivierung bzw. Sterilisierung der bodeneigenen Mikroflora wurden die mit Boden gefüllten Mikrotiterplatten pasteurisiert. Hierzu wurden sie zweimal bei 85°C für jeweils 10 min in ein Wasserbad gestellt.

### Konservierung und Kultivierung der Testbakterien

### Gefriertrocknung

Eine erfolgreiche Gefriertrocknung von Bakterien ist dann erreicht, wenn die Bakterienzellen ihre Aktivität nicht einbüßen bzw. die Zellen nicht zerstört werden. Wesentlichen Einfluss darauf haben Schutzstoffe bzw. Stabilisatoren. Dies sind zum einen anorganische Ionen zur Einstellung der Isotonie, die beim Einfrieren eine Schädigung der Zelle durch Anreicherung von Elektrolyten verhindern. Des weiteren Puffersubstanzen zur Stabilisierung des pH-Wertes und kryoprotektive Gerüstsubstanzen, die Wassermoleküle ersetzen, welche beim Einfrieren aus deren Hydrathülle entfernt werden.

Die zu gefriertrocknende Bakteriensuspension wurde hergestellt, indem Schrägagarkulturen (Caso-Agar) mit Hilfe einer Impföse mit der Stammkultur beimpft wurden. Diese Kulturen wurden bei 30°C 24 Stunden lang inkubiert. Da eine hohe Zelldichte erhalten werden sollte, wurden ungefähr 10 gut gewachsene Kulturen mit 10 ml Nährmedium B ein bis zweimal abgeschlemmt. Dadurch wurde eine dichte Bakteriensuspension erhalten.

Bei der hier angewandten Gefriertrocknung wurde zunächst die Schutzlösung (20% Magermilchpuver, 5% Myo-Inosit) lyophilisiert. Hierzu wurden jeweils 0,5 ml der Schutzlösung in kleine HPLC-Vials abgefüllt, zunächst bei - 80°C eingefroren und anschließend an der Gefriertrocknungsanlage getrocknet. Im zweiten Schritt wurden dann die 0,5 ml der Bakteriensuspension auf das Schutzlösungs-Lyophilisat gegeben und nochmals für etwa zwei Stunden bei -80°C eingefroren und dann an der Lyophille getrocknet. Die fertigen gefriergetrockneten Bakterien wurden bei -20°C gelagert.

### Reaktivierung der gefriergetrockneten Bakterien

Zur Reaktivierung der gefriergetrockneten Zellen wurden 0,5 ml kaltes steriles vollentionisiertes Wasser auf das Lyophilisat gegeben und für ungefähr 15 Minuten in den Kühlschrank gestellt.

Für die Herstellung des Inokulums wurde die reaktivierte Bakteriensuspension in 25 ml Nährmedium B gegeben, welches auf 30°C temperiert wurde.

### Testdurchführung

### Inkubation

Je Vertiefung wurden 0,4 ml Inokulum auf den pasteurisierten Boden gegeben und für eine Kontaktzeit von zwei Stunden im Brutschrank bei 30°C im Dunkeln auf einem HorizontalSchüttler inkubiert. Zur Überprüfung der Inaktivierung der bodeneigenen Mikroflora wurde in eine Reihe an Stelle von Inokulum Nährmedium B gegeben.

### Reaktion

Nach dieser Kontaktzeit wurde je Vertiefung 0,8 ml Resazurin dazu gegeben und sofort die relative Fluoreszenz des gebildeten Resorufins bei einer Wellenlänge von 590 nm nach Anregung mit Licht einer Wellenlänge von 535 nm gemessen. Diese Messung wurde im Abstand von 15 Minuten eine Stunde lang wiederholt. Die Zunahme des Resorufins ist proportional zur Dehydrogenaseaktivität der applizierten Testbakterien und somit ein Maß der Toxizität des untersuchten Bodens.

### Ermittlung der Hemmung der Dehydrogenaseaktivität

Zur Ermittlung der durch Schadstoffe hervorgerufenen Hemmung der Dehydrogenaseaktivität wurde die Steigung der Resorufin-Zunahme im linearen Zeitraum von 15 bis 45 Minuten für die unterschiedlich stark kontaminierten Böden berechnet und diese auf die Steigung des unkontaminierten Bodens bezogen.

### Ergebnisse der Chemikalienbewertung

Die hier ermittelten Ergebnisse zur Empfindlichkeit stimmen mit den Ergebnissen eines Ringversuches überein, in dem bei 600 mg/kg eine 58%ige Hemmung ermittelt wurde. Die in einer 1995 durchgeführten Untersuchung mit Bacillus cereus erhaltenen Ergebnisse ergaben bei 400 mg/kg BAC eine 50%ige Hemmung der Dehydrogenaseaktivität, was ebenfalls für die hier ermittelten Ergebnisse spricht [RÖNNPAGEL, 1995] (s. Abb. 2).

Die folgende Gegenüberstellung zeigt deutlich, dass eine erhebliche Miniaturisierung der eingesetzten Volumina erfindungsgemäß erzielt wird:

**Tabelle 2: Vergleich der Volumina klassisches Verfahren / erfindungsgemäß miniaturisierter Ansatz**

| | **Klassisches Verfahren** | **Miniaturisierter Ansatz** |
|---|---|---|
| Probe (g) | 3,0 | 0,6 |
| Wasser (ml) | 3,0 | 0,6 |
| Inoculum (ml) | 2,0 | 0,4 |
| Resazurinlösung (ml) | 4,0 | 0,8 |
| **Gesamtvolumen** | 3 g + 9 ml | 0,6 g + 1,8 ml |

In der nachfolgenden Gegenüberstellung werden die Bearbeitungszeit incl. Inkubationszeiten vom Stand der Technik gegenüber dem miniaturisierten, erfindungsgemäßen Ansatz aufgezeigt:

**Tabelle 3: Bearbeitungszeit für 5 Proben**

| | **Klassisches Verfahren** **(h)** | **Miniaturisierter Ansatz** **(h)** |
|---|---|---|
| Vorkultur I | 16 | entfällt |
| Vorkultur II | 2 | entfällt |
| Reaktivierung der Bakterien | entfällt | 0,25 |
| Ansatz | 2,7 | 0,5 |
| Kontaktzeit | 2 | 2 |
| Reaktionszeit (Enzymatik) | 0,75 | 0,75 |
| Zentrifugation/Filtration | 0,5 | entfällt |
| Messung | 0,25 | 0,1 |
| **(Gesamtzeit)** | **24,2** | **3,6** |

## Patentansprüche

1. Verfahren zum Bestimmen der ökotoxikologischen Wirkung feststoffadsorbierter und/oder in Wasser gelöster Schadstoffe in einer Matrix mittels Zusatz reaktivierter, vorher gefriergetrockneter Bakterien als Inokulum, Inkubation desselben und Ermittlung der Hemmung ihrer Dehydrogenaseaktivität, die über den Umsatz eines Redoxfarbstoffes bestimmt wird,
**dadurch gekennzeichnet, dass**
der Testansatz in Mikrotiterplatten durchgeführt wird, wobei die Matrix in einer Menge von < 1g eingegeben wird, und wobei die Inkubation in einem Schüttler/Mischer durchgeführt wird, und wobei die Zunahme des reduzierten Redox-Stoffes fluorimetrisch direkt in der Mikrotiterplatte erfasst wird, und dass sämtliche Verfahrensschritte in einer Zeitspanne < 8 Std. durchgeführt werden.

2. Verfahren nach Anspruch 1, **dadurch gekennzeichnet, dass** eine Kinetik der relativen Fluoreszenz aufgenommen wird.

3. Verfahren nach einem der Ansprüche 1 oder 2, **dadurch gekennzeichnet, dass** die Bakterien mit einer Reaktivierungslösung in Form von sterilem vollentionisiertem Wasser oder einer sterilen 2%igen Glucoselösung oder einem sterilen Nährmedium reaktiviert werden.

4. Verfahren nach einer oder mehreren der Ansprüche 1 bis 3, **dadurch gekennzeichnet, dass** Bakterien der Gattung Arthrobacter globiformis Bacillus cereus, Corynebacterium sp., Escherichia coli, Pseudomonas aeruginosa, Pseudomonas putida und/oder Salmonella sp. eingesetzt werden.

5. Verfahren nach einem oder mehreren der Ansprüche 1 bis 4, **dadurch gekennzeichnet, dass** als Redoxfarbstoff zu Resorufin reduziertes Resazurin oder Tetrazoliumsalz: 5-Cyano2,3-ditolyltetrazoliumchlorid (CTC) oder Triphenyltetrazoliumchlorid (TTC) oder Iodonitrophenlytetrazolliumchlorid (INT) eingesetzt wird.

6. Verfahren nach einem oder mehreren der Ansprüche 1 bis 5, **dadurch gekennzeichnet, dass** die Inkubationen im Horizontalschüttler durchgeführt wird.

7. Verfahren nach einem oder mehreren der Ansprüche 1 bis 6, **dadurch gekennzeichnet, dass** die Gesamttestzeit 6 Std. oder geringer beträgt.

8. Verfahren nach einem oder mehreren der vorstehenden Ansprüche, **dadurch gekennzeichnet, dass** die Matrix aus Boden, Sediment, Baustoffen und/oder Abfall jeglicher Art, sowie Eluaten hieraus, besteht.

9. Verfahren nach einem oder mehreren der vorstehenden Ansprüche, **dadurch gekennzeichnet, dass** Schadstoffe in Form von Umweltproben oder als zu bewertende Chemikalien mit einer Nachweisgrenze bei Umweltproben zwischen 10 und 50% Hemmung bezogen auf eine unkontaminierte Kontrolle, insbesondere zwischen 20% und 40% Hemmung, und bei einer Chemikalienbewertung über die Angabe des EC₅₀ (die mittlere Konzentration eines Stoffes in der Matrix, bei der 50% des Effekts innerhalb der Versuchsdauer auftritt) und NOEC (No observed effect concentration) bestimmt werden.

10. Verfahren nach einem oder mehreren der vorstehenden Ansprüche 1 bis 9, **gekennzeichnet durch** einen Konzentrationsbereich des Inokulums von 1,0 x 10⁵ - 1,0 x 10⁹, insbesondere 5,0 x 10⁷ - 5,0 x 10⁸.

11. Testkit zum Durchführen des Verfahrens in einem Labor nach einem oder mehreren der vorstehenden Ansprüche 1 bis 10, **gekennzeichnet durch**:
eine oder mehrere Mikrotiterplatten, gefriergetrocknete Bakterien (Inokulum), eine Reaktivierungslösung und eine Redoxfarbstofflösung unter Einsatz eines im Labor vorhandenen Schüttlers oder Mischers und eines Fluoreszenzmeßgerätes mit Einrichtung zum direktem Messen von Lösungen in Mikrotiterplatten.

12. Testkit nach dem vorstehenden Anspruch 11, **gekennzeichnet durch** folgende Abmessungen bzw. Volumina
der Mikrotiterplatten: 12,5 x 8,5 cm mit 24 Vertiefungen des Inokulums: 0,4-0,8 ml/Vertiefung
der Redoxfarbstofflösung: 0,8-1,6 ml/Vertiefung
